# EUROPEAN PATENT SPECIFICATION

(11) **EP 0 295 777 B1**
(45) Date of publication and mention of the grant of the patent: **21.07.1993**
(21) Application number: 88304199.8
(22) Date of filing: 09.05.1988
(51) Int. Cl.: A61L 15/16

(54) **Wound dressing**
Wundverband
Pansement pour blessure

(30) Priority: 16.06.1987 JP 91310/87
(43) Date of publication of application: 21.12.1988
(73) Proprietor: JAPAN GORE-TEX, INC., Setagaya-ku Tokyo 156 (JP)
(72) Inventor: Sakai, Mari, Akoda Okayama-shi 703 (JP)
(74) Representative: Klunker . Schmitt-Nilson . Hirsch

(56) References cited:
- EP-A- 0 068 777
- EP-A- 0 171 254
- DE-A- 1 906 159

## Description

The present invention concerns a wound dressing, i.e. a wound-covering material.

When dressing a wound it is desirable (a) to ensure antibacterial barrier properties and waterproof properties in the covering material which is applied to cuts, burns and other wound in order to protect such wounds: (b) to ensure acceptable compatibility with the wound; (c) to maintain air and water vapour permeability; and (d) to prevent invasion of the covering material by body tissues so that stripping of the covering material from the wound is facilitated.

The present invention may be applied to covering materials for cuts and other wounds including artificial skin and other biocompatible materials.

Generally, woven or knitted fabrics, papers and films coated with pharmocologically effective compounds have been used in the past as materials for covering wounds. In addition, biological materials such as collagen films, chitin films, fibrin films and pigskin have also been used as such covering materials. In addition, soft synthetic products such as silicone rubber films have been used to some extent.

In the case of the above-mentioned conventional covering materials, invasion of the wound by bacteria and water from the outside generally cannot be completely prevented. Furthermore, materials which can prevent invasion of the wound do not generally possess air permeability or moisture vapour permeability. As a result, the affected area may become inflamed, thus retarding the healing process and subjecting the patient to discomfort. Moreover, such wound covering materials may adhere to the wound, and thus further injure the wound every time the covering material is changed. The patient using the covering material is thus subjected to additional suffering.

EP-A-0068777 discloses a composite wound dressing of a polytetrafluoroethylene fibril matrix, hydrophilic absorptive particles enmeshed in the matrix which particles may include chitin particles (claim 2), and a partially occlusive film coated on one surface of the matrix. This is a three-component composite which includes chitin particles, not a chitin film. Although the "occlusive film" should have a controlled permeability or porosity, it acts as a barrier blocking the free evaporation of moisture. The three component structure is difficult to manufacture and expensive.

According to the present invention there is provided a wound dressing comprising a layer of chitin affixed to a layer of porous, expanded polytetrafluoroethylene (PTFE).

The chitin may be laminated to the PTFE or it may penetrate into and be impregnated in the porous PTFE layer. The dressing preferably has a pressure sensitive adhesive applied in a dot pattern on its surface, thereby providing means to apply and secure the dressing over a wound. The porous, expanded PTFE preferably has a microstructure of nodes interconnected by fibrils wherein fibril lengths preferably range from 0.01 to 1.0 micrometers. A layer of a water-absorbing macromolecular material may be interposed between the chitin layer and the PTFE layer. The water-absorbing macromolecular material preferably is polyvinyl alcohol or an acrylic acid graft-polymerized starch which will absorb blood and other liquids.

The invention will now be particularly described by way of example with reference to the accompanying drawings in which:-
Figure 1 is a perspective view, partly in cross-section, of one embodiment of the wound dressing according to the invention;
Figure 2 is a cross-sectional view of another embodiment of the invention, and
Figure 3 is a cross-sectional view of still another embodiment of the invention.

A wound dressing is provided comprising a layer of chitin affixed to a layer of porous, expanded polytetrafluoroethylene (PTFE). The chitin may be laminated to the PTFE or it may penetrate and be impregnated in the porous PTFE layer. The composite preferably has a pressure sensitive adhesive applied in a dot pattern on its surface to enable application and securing of the dressing over a wound.

The chitin layer decomposes and is absorbed by the wound. The abovementioned porous, expanded polytetrafluoroethylene film is applied so that it fits appropriately over the chitin and over the area of the wound.

The micropores of the PTFE film ensure air and water vapour permeability. Moreover, the micropores prevent invasion of the wound by bacteria and dust, and act in combination with the water-repellent nature of the material itself to provide waterproof characteristics. Furthermore, the covering material can easily be peeled from the wound even in cases where all of the chitin has been decomposed and absorbed. The porous, expanded PTFE material and its method of manufacture are disclosed in U.S. Patent 3,953,566.

The following is a description of examples of the present invention with reference to the accompanying drawings.

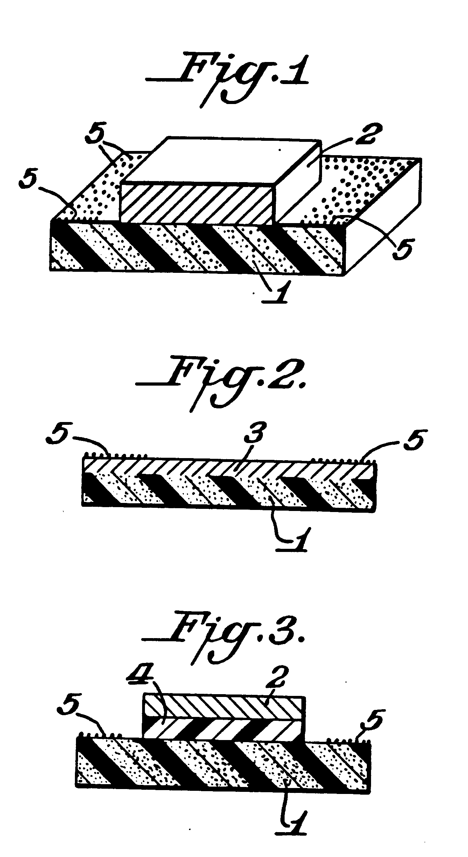

First, in regard to the manner of application of the chitin 2 to the porous, expanded polytetrafluoroethylene film 1, the chitin 2 may be laminated with the porous film as shown in Figure 1 or a chitin-impregnated layer 3 may be formed as shown in Figure 2. The chitin layer preferably is affixed to the PTFE layer as shown in Figure 1 as follows: (1) the chitin layer is laminated on to the PTFE layer by an adhesive which is harmless to living body tissue, for example, as a chitin solution; (2) this chitin solution is applied as a coating on to the PTFE layer; (3) the chitin solution may be prepared by using a solvent for chitin such as a mixture of lithium chloride and dimethyl acetamide or a mixture of lithium chloride and N-methylpyrrolidone. It is preferable to dissolve chitin into the solvent containing lithium chloride of over 5 weight percent so as to obtain a solution containing chitin of 0.3 to 10 weight percent; and (4) after the chitin solution is coated on to the PTFE film, it is coagulated by immersing it into a coagulating liquid, preferably water, methyl alcohol, ethyl alcohol or acetone. After coagulation, the laminate is washed with water.

Another method is to acetylate chitosan after coating into chitin. Chitosan produced by deacetylation of chitin is readily soluble in acid. It is acetylated after forming it into a membrane.

An example is as follows: (a) chitosan is dissolved into a water solution containing acetic acid of 10 weight percent so as to obtain a solution containing chitosan of 5 weight percent; (b) the solution obtained containing chitosan is diluted with methyl alcohol to three times its volume; (c) the diluted solution obtained is coated on to the PTFE layer and left to dry; (d) after drying, the composite is swollen by immersing it into methyl alcohol containing water of 20 weight percent for about ten minutes. Then acetic anhydride (2 to 100 mole per one mole of glucosamine) is added and it is left as it is for 30 minutes; (e) the composite is immersed into a water solution containing 2 percent NaOH for 30 minutes; and (f) the composite is then washed with water and left to dry.

Chitin dissolved into a solvent may be impregnated into a PTFE membrane, as shown in Figure 2, by applying pressure, using vacuum techniques or employing supersonic waves.

The chitin layer 2 may be formed on the aforementioned film 1 with a water-absorbing macromolecular material 4 such as polyvinylalcohol or an acrylic acid graft-polymerized starch which will absorb blood and other liquids interposed between layer 2 and film 1, as shown in Figure 3.

In all cases, a pressure sensitive adhesive can be applied, preferably in a dot pattern, to the peripheral areas of the porous, expanded polytetrafluoroethylene film 1 so that the film can be caused to adhere to the area surrounding the wound that it is to cover. Furthermore, therapeutic drugs or antiobiotics may be included in the chitin film 2 or chitin-impregnated layer 3. Moreover, the chitin 2 may have a sponge-form or nonwoven-fabric-form porous structure.

The abovementioned porous, expanded polytetrafluoroethylene film is obtained by fibrilizing a polytetrafluoroethylene film by rolling or drawing as disclosed in U.S. Patent 3,953,566, so that a structure is formed in which numerous micronodes are connected by countless fibrils. By making the pore size (fibril length of this structure 0.01 to 1 micron, it is possible to effectively prevent the permeation of the film by bodily fluids or body tissues. As a result, the covering material can easily be peeled from the wound to which it is applied.

The chitin is a biotechnological material. As a tissue fibre component found in crustaceans, it fulfills the biological functions of both collagen in higher animal tissues and cellulose in higher plant tissues, and has a desirable compatibility with living cells. When this chitin is applied to a wound, it is appropriately decomposed and absorbed. By adjusting the rate of absorption, it is possible to prevent sticking to the wound and a desirable healing effect is obtained. Even when all of the chitin is absorbed, the covering material can quickly be peeled from the wound by means of the porous, expanded polytetrafluoroethylene film 1 and replaced.

As was described above, the covering material of the present invention, which consists principally of a porous, expanded polytetrafluoroethylene film 1, fits effectively over the area of the wound. Furthermore, as a result of its porous structure, this film is both air permeable and moisture-vapour-permeable. In addition, as a result of the small pores of the film and the liquid-water-repellent nature of the material itself, the film has waterproof characteristics and thus prevents the passage of bodily fluids. Similarly, the film completely prevents invasion of the wound by bacteria or small dust particles. Accordingly, the present invention provides a comfortable and desirable covering material for cuts and other wounds, which possesses waterproof characteristics and antibacterial barrier properties.

The chitin is generally obtained as a semitransparent or nearly transparent material. This chitin has a uniform thickness as a result of being applied to the porous, expanded polytetrafluoroethylene film. Furthermore, this material has an appropriate tensile strength and is therefore suitable for handling. In particular, since this material can be formed into an extremely thin film, it is easy to fit over the surface of the body, and since it is not bulky, the covering material can be applied to the fingers and will not catch on other objects as work is being performed. Since the covering material has waterproof characteristics as described above, various types of work such as cooking or laundering can be smoothly performed by a person wearing this covering material over a wound.

As described above, the present invention can provide a covering material which fits appropriately over the area of a wound and which has air and moisture-vapour permeability so that the wearer feels no substantial uncomfortable dampness. Moreover, this material has waterporoof characteristics and antibacterial barrier properties, and fits appropriately on the surface of the body, so that a superior therapeutic effect is obtained. Following absorption of the chitin, the material can easily be peeled away from the wound without causing the wearer any substantial discomfort.

## Claims

1. A wound dressing characterised by a layer of chitin affixed to a layer of porous, expanded polytetrafluoroethylene (PTFE).

2. A dressing according to claim 1 characterised in that the chitin is laminated to the PTFE.

3. A dressing according to claim 1 characterised in that the chitin penetrates into and is impregnated in the porous PTFE layer.

4. A dressing according to any preceding claim characterised by a pressure sensitive adhesive applied in a dot pattern on its surface, thereby providing means to apply and secure said dressing over a wound.

5. A dressing according to any preceding claim characterised in that said porous, expanded PTFE has a microstructure of nodes interconnected by fibrils wherein fibril lengths range from 0.91 to 1.0 micrometers.

6. A dressing according to any preceding claim characterised in that a layer of water-absorbing macromolecular material is interposed between said chitin layer and said PTFE layer.

7. A dressing according to claim 6 characterised in that said water-absorbing macromolecular material is polyvinylalcohol.

8. A dressing according to claim 6 characterised in that said water-absorbing macromolecular material is an acrylic acid graft-polymerized starch.

## Patentansprüche

1. Wundverband, **gekennzeichnet durch** eine Chitinschicht, die an einer Schicht aus porösem, expandiertem Polytetrafluorethylen (PTFE) befestigt ist.

2. Verband nach Anspruch 1, **dadurch gekennzeichnet,** daß das Chitin auf das PTFE auflaminiert ist.

3. Verband nach Anspruch 1, **dadurch gekennzeichnet,** daß das Chitin in die poröse PTFE-Schicht eindringt und sie imprägniert.

4. Verband nach irgendeinem vorhergehenden Anspruch, **gekennzeichnet durch** einen druckempfindlichen Klebstoff, der in einem Punktmuster auf seine Oberfläche aufgebracht ist und dadurch Mittel zum Anbringen und Befestigen des Verbands über einer Wunde bildet.

5. Verband nach irgendeinem vorhergehenden Anspruch, **dadurch gekennzeichnet,** daß das poröse, expandierte PTFE eine Mikrostruktur aus Knötchen aufweist, die durch Fibrillen miteinander verbunden sind, wobei die Längen der Fibrillen von 0,91 bis 1,0 µm reichen.

6. Verband nach irgendeinem vorhergehenden Anspruch, **dadurch gekennzeichnet,** daß eine Schicht aus wasserabsorbierendem, makromolekularem Stoff zwischen der Chitinschicht und der PTFE-Schicht angeordnet ist.

7. Verband nach Anspruch 6, **dadurch gekennzeichnet,** daß der wasserabsorbierende, makromolekulare Stoff Polyvinylalkohol ist.

8. Verband nach Anspruch 6, **dadurch gekennzeichnet,** daß der wasserabsorbierende makromolekulare Stoff eine acrylsäure-pfropfpolymerisierte Stärke ist.

## Revendications

1. Pansement de plaie caractérisé par une couche de chitine fixée sur une couche de polytétrafluoroéthylène (PTFE) expansé poreux.

2. Pansement selon la revendication 1 caractérisé en ce que la chitine est laminée sur le PTFE.

3. Pansement selon la revendication 1 caractérisé en ce que la chitine pénètre dans la couche poreuse de PTFE et l'imprègne.

4. Pansement selon une quelconque des revendications précédentes, caractérisé en ce qu'un auto-adhésif est appliqué à sa surface, par points, de façon à fournir un moyen d'appliquer et maintenir ledit pansement sur une plaie.

5. Pansement selon une quelconque des revendications précédentes caractérisé en ce que ledit PTFE expansé poreux a une microstructure de noeuds reliés entre eux par des fibrilles, fibrilles dont la longueur va de 0,91 à 1,0 micromètres.

6. Pansement selon une quelconque des revendications précédentes caractérisé en ce qu'une couche de matériau moléculaire absorbeur d'eau est interposée entre ladite couche de chitine et ladite couche de PTFE.

7. Pansement selon la revendication 6 caractérisé en ce que ledit matériau macromoléculaire absorbeur d'eau est de l'alcool polyvinylique.

8. Pansement selon la revendication 6 caractérisé en ce que ledit matériau macromoléculaire absorbeur d'eau est un amidon polymérisé greffé à de l'acide acrylique.
